# EUROPEAN PATENT APPLICATION

(11) **EP 4 382 201 A1**
(43) Date of publication of application: **12.06.2024**
(21) Application number: 21952572.2
(22) Date of filing: 16.11.2021
(51) Int. Cl.: B01J 23/755, B01J 27/185, C10G 3/00

(54) **PHOSPHORUS-DOPED NICKEL ALUMINUM OXIDE, AND PREPARATION METHOD THEREFOR AND USE THEREOF**

(30) Priority: 03.08.2021 CN 202110886367
(71) Applicant: Qingdao Institute of Bioenergy and Bioprocess Technology, Chinese Academy of Sciences, Qingdao, Shandong 266101 (CN)
(72) Inventor: WANG, Guanghui, Qingdao, Shandong 266101 (CN); LI, Dechang, Qingdao, Shandong 266101 (CN); PAN, Zhengyi, Qingdao, Shandong 266101 (CN); ZHANG, Shan, Qingdao, Shandong 266101 (CN)
(74) Representative: Seyer & Nobbe Patentanwälte PartmbB
(86) International application number: PCT/CN2021/130970
(87) International publication number: WO 2023/010709

(57) **Abstract**

The present invention relates to the technical field of new catalytic materials, and specifically relates to a phosphorus-doped nickel-aluminium oxide, its preparation method and the application thereof. Said preparation method comprises: the nickel-aluminium-based layered double hydroxide is subjected to high-temperature aerobic calcination to obtain nickel-aluminium oxide, the nickel-aluminium oxide obtained thereby is mixed with a phosphorus source and heated in an inert gas atmosphere or in vacuum conditions to dope phosphorus into the nickel-aluminium oxide, whereby the final phosphorus-doped nickel-aluminium oxide is obtained. In the present invention the Ni-Al interactions are constructed by subjecting the nickel-aluminium based layered double hydroxides to aerobic calcination at high temperature. The Ni-P interactions are constructed by doping P into nickel-aluminium oxides. Thus, the synergistic interactions of Ni-Al and Ni-P achieve regulating the electron density around the P-active metals, wherein the active metal is in an intermediate phase between metal and metal phosphide, which suppresses the factors leading to deactivation of the catalyst, such as metal agglomeration, carbon deposition, and phase transformation, demonstrating excellent catalytic activity, selectivity and stability.

## Description

### Technical Field

The present invention relates to the technical field of novel catalytic materials, and specifically the present invention relates to a phosphorus-doped nickel-aluminium oxide, its preparation method and the application thereof.

### Background Art

The disclosure of the information in this background art merely serves the purpose of helping to understand the general concept of the invention and is not necessarily to be taken as an admission or in any way implying that the information constitutes prior art that is already well known to those of ordinary skill in the art.

Hydrodeoxygenation (HDO) is an important class of catalytic reaction processes that typically occur in the production of biofuels or chemicals from oxygen-containing compounds, such as biomass-based molecules. HDO reactions generally take place at high temperatures and high pressures, which may easily cause agglomeration of active metals and deposition of carbon on the surface of catalysts, thereby detrimentally affecting the catalytic activity and stability. In addition, during the catalytic C-O/C=O deoxygenation, it is thermodynamically easier for hydrocracking reactions of C=C, C-C and C-H to occur. Said reactions will detrimentally affect the deoxygenation efficiency and form excessive hydrogenation by-products. Therefore, current research as well as industrial applications in the relevant field is focusing on developing cost-effective, highly efficient and stable catalysts having improved efficiency and selectivity for HDO reactions.

Chen et al. (Renew. Sust. Energ. Rev. 2019, 101, 568-589) summarizes in detail the commonly used catalysts for oil HDO in a review. The noble metal catalysts, such as Pd, Pt, Ru and Rh, show high catalytic activity and high reaction efficiency. However, such catalysts are expensive and easily deactivated, which deficiencies limit their large-scale applications. The research on non-precious metal catalysts is focusing on Ni, Co, Cu and Fe catalysts. Although said non-precious metal catalysts are readily available, they have the drawback of low reaction efficiencies and usually require relatively high reaction temperatures (350-450 °C) and pressures (5-15 MPa), and thus are susceptible to C-C bond cracking and carbon deposition etc. Sulphides commonly used in the petrochemical industry have also demonstrated some catalytic activity for HDO reactions of oils, but the initial activity of sulphide catalysts is low and they are susceptible to loss of sulphur during the reaction, leading to catalyst deactivation and environmental pollution. Metal phosphides are a class of promising non-precious metal catalysts, wherein the phosphorus can improve the metal center's affinity for oxygen-containing functional groups by changing the electronic structure around the metal centers, thus avoids catalytic cracking at the metal centers and demonstrates high deoxygenation selectivity. However, the inventors find that in previous studies, most phosphides catalysts are susceptible to carbon deposition or phase transformation leading to catalyst deactivation. Thus, the overall catalytic performance is not satisfactory and not suitable for long term applications.

### Content of the invention

To overcome the above-mentioned deficiencies in the prior art, the present invention provides a phosphorus-doped nickel-aluminium oxide, its preparation method and the use thereof, which solve the technical problem that conventional phosphide catalysts are susceptible to catalyst deactivation due to carbon deposition or phase transformation. Distinguished from the conventional metal phosphides in the prior art, the material prepared according to the inventive method achieves regulation of the electron density around the catalytically active metals by constructing Ni-Al and Ni-P interactions while maintaining the metal in the oxide phase. Thus, the active metals are kept in the intermediate phase between metal and metal phosphides, which suppresses the factors leading to deactivation such as metal agglomeration, carbon deposition and phase transformation, and demonstrates excellent catalytic activity, selectivity and stability.

In order to achieve the above objective, the first aspect of the present invention is to provide a method for the preparation of phosphorus-doped nickel-aluminum oxide. In more detail, a nickel-aluminum-based layered double hydroxide is calcined aerobically at high temperature to provide a nickel-aluminum oxide, and then the nickel-aluminum oxide is mixed with a phosphorus source and heated under the protection of an inert gas or in a vacuum to dope the phosphorus into the nickel-aluminum oxide. Thus, the phosphorus-doped nickel-aluminum oxide is obtained.

A second aspect of the present invention is to provide a phosphorus-doped nickel- aluminum oxide obtained by the above preparation method.

A third aspect of the present invention is to provide a phosphorus-doped nickel-aluminum oxide as described above for use in catalytic hydrodeoxygenation reactions.

One or more embodiments of the present invention have at least the following advantages:
(1) In the present invention Ni-Al interactions are constructed by subjecting nickel-aluminum-based layered double hydroxides to high-temperature aerobic calcination. Ni-P interactions are built by doping P into nickel-aluminum oxides. The synergistic interactions between Ni-Al and Ni-P achieve regulation of the electrons around the P-active metals. Thus, the active metals are maintained in the intermediate phase between metal and metal phosphide, which suppresses the factors leading to deactivation such as metal agglomeration, carbon deposition and phase transformation, and demonstrates excellent catalytic activity, selectivity and stability.
(2) The phosphorus-doped nickel-aluminum oxide prepared according to the present invention shows excellent catalytic performance in hydrodeoxygenation reactions, the conversion of the feedstock reaches more than 95%, the hydrodeoxygenation efficiency reaches more than 90% with less cracking products produced. After continuously operating under the optimized conditions for more than 500 hours the catalyst does not show any appreciable deactivation indicating its superior stability and excellent prospect for industrial applications.
(3) The phosphorus-doped nickel-aluminum oxide according to the present invention can be prepared from cheap and easily available raw materials by simple method according to the present invention. The inventive method can be easily scaled up for industrial production.
(4) The phosphorus-doped nickel-aluminum oxide prepared according to the present invention is universally applicable for catalyzing the hydrodeoxygenation reactions of various oxygen-containing compounds.

### Description of the Drawings

The figures accompanying the specification forming part of the present invention serve for better understanding the invention, and the embodiments of the invention and their descriptions serve for explaining the invention without constituting an undue limitation of the invention.

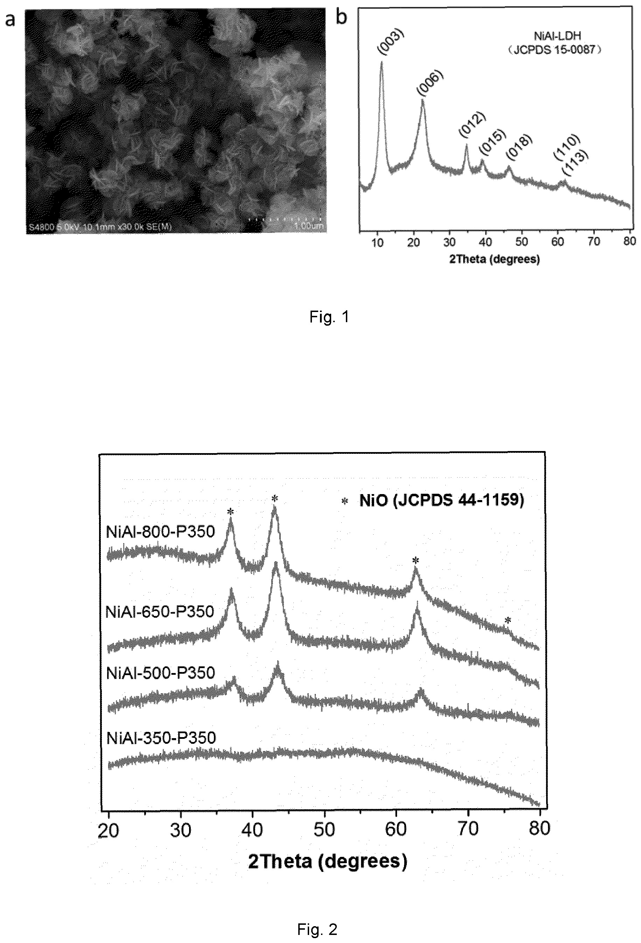

Figure 1 shows a SEM image a) and an XRD spectrum b) of the layered double hydroxide precursor having a Ni : Al ratio of 3 : 1 used in Examples 1-3 of the present invention and comparative Examples 1-3.
Figure 2 shows the XRD spectra of samples in Examples 1-3 according to the present invention and Comparative Example 2 obtained by calcining at various temperatures followed by phosphorizing at 350 °C.

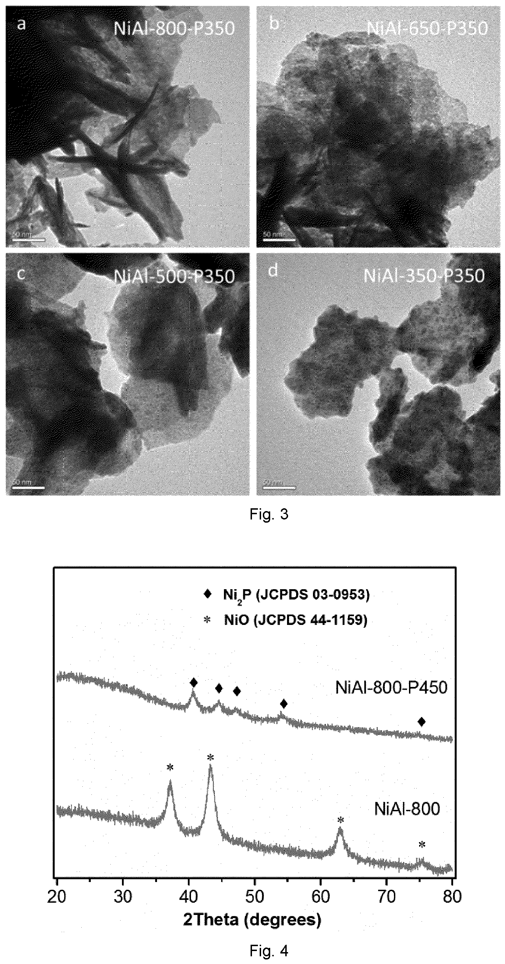

Figure 3 shows the TEM images of samples in Examples 1-3 according to the present invention and Comparative Example 2 obtained by calcining at various temperatures followed by phosphorizing at 350 °C.
Figure 4 shows the XRD spectra of Comparative Examples 1 and 3.
Figure 5 shows the results of the hydrodeoxygenation reaction of methyl laurate catalyzed by NiAI-800-P350 in Example 4.
Figure 6 shows the results of the catalytic performance of NiAI-800-P350 at different reaction temperatures in Example 5.
Figure 7 shows the results of the hydrodeoxygenation reaction of methyl laurate catalyzed by NiAI-650-P350 and NiAI-500-P350 in Example 6.
Fig. 8 shows the results of the hydrodeoxygenation reaction of methyl laurate catalyzed by phosphorus-doped NiAl-oxide obtained in Comparative Examples 1-3 (i.e. the results in Examples 4-6).

### Detailed Description of Certain Embodiments

It should be noted that the following detailed descriptions are all exemplary and are intended to provide further illustration of the invention. Unless otherwise indicated, all technical and scientific terms used herein have the same meaning as commonly understood by those of ordinary skill in the art to which the present invention belongs.

It is to be noted that the terms used herein are intended to describe specific embodiments only and are not intended to limit the exemplary embodiments according to the present invention. As used herein, unless the context clearly indicates otherwise, a singular form is also intended to include the plural form, and it is furthermore to be understood that when the terms "comprising" and/or "including" are used in this specification, they indicate the presence of features, steps, operations, devices, components and/or combinations thereof.

As mentioned in the background art, metal phosphides are promising non-precious metal catalysts, wherein P alters the electronic structure of the metal centers, and thus increases the affinity of the metal centers for oxygen-containing functional groups and thus, avoids catalytic cracking on the active metal sites, demonstrating high selectivity towards deoxygenation reaction. However, most phosphide catalysts are susceptible to carbon deposition or phase transformation leading to deactivation of the catalyst and making the catalytic performance unstable and hence not suitable for long term applications.

In order to solve the above-mentioned technical problems, the first aspect of the present invention is to provide a method for the preparation of phosphorus-doped nickel-aluminium oxides. In detail, nickel-aluminium-based layered double hydroxide compounds are calcined aerobically at high temperatures to obtain nickel-aluminium oxides, and then nickel-aluminium oxides are mixed with a phosphorus source and heated under the protection of an inert gas or in a vacuum to dope phosphorus into the nickel-aluminium oxides, and finally phosphorus-doped nickel-aluminium oxides are obtained.

Metal phosphides are highly efficient non-precious metal catalysts. The P can improve the affinity for oxygen-containing functional groups by altering the electronic structure around the metal center and avoids catalytic cracking on the active metal sites. However, there is a general problem of carbon deposition or phase transformation resulting in catalyst deactivation during the catalytic process. The inventors find that the reason for this phenomenon is the presence of the nickel phosphide phase. In order to take advantage of the electronic structure of the P atoms and to avoid the problem of catalyst deactivation, it is found that in the present invention loading the P in the form of doping while not in the form of nickel phosphide on the metal oxide ensures taking advantage of the P, while not bringing about catalyst deactivation due to carbon deposition or phase transformation.

To achieve the above objective, nickel-aluminium oxides are prepared by high temperature aerobic calcination of nickel-aluminium based layered double hydroxides according to the present invention. The so-obtained nickel-aluminium oxides involve Ni-Al interactions, and thus the Ni-species has a relatively high reduction energy barrier, which helps to keep the nickel as phosphorus doped metal oxide after phosphorization without forming nickel phosphide phase. At the same time, in the present invention the nickel-aluminium oxides and the phosphorus source are treated by heating, thus the P is directly doped into the nickel-aluminium oxides further preventing the formation of a nickel phosphide phase, while successfully constructing Ni-P interactions. By constructing Ni-Al and Ni-P interactions, the present invention achieves the regulation of the electron density around the P-active metals. Thus, the active metals are maintained in the intermediate phase between metal and phosphide, which suppresses the factors leading to deactivation such as metal agglomeration, carbon deposition and phase transformation, and demonstrates excellent catalytic activity, selectivity and stability.

As mentioned hereinabove, the Ni-Al interaction has a substantial influence on whether the nickel phosphide phase is present in the final product. Said Ni-Al interaction is dependent on the molar ratio of Ni to Al, which, in an appropriate ratio, enables a more stable Ni-Al interaction to be established. As a preferred embodiment, the molar ratio of Ni to Al in said nickel-aluminium based layered double hydroxide is 5:1-1:5, preferably 4:1-2:1.

Said nickel-aluminium-based layered double hydroxides are synthesized mainly to ensure stable Ni-Al interactions. The method of synthesis is not specially limited and depending on the need, they can be selected from any one of the methods of hydrothermal synthesis, co-precipitation, ion exchange, and calcination-recovery, preferably hydrothermal synthesis.

In one or more embodiments of the present invention, the process of preparing nickel-aluminium oxides by hydrothermal synthesis is specified as follows: the nickel precursor, aluminium precursor and alkali precursor are heated in an aqueous solution in a hydrothermal reactor, washed and dried to obtain a nickel-aluminium-based layered double hydroxide;

Furthermore, said nickel precursor and aluminium precursor are selected from one or more of nitrate, acetate, halide, sulphate, respectively, preferably nickel nitrate and aluminium nitrate. The alkali precursor is selected from one or more of urea, hexamethylene tetramine, sodium bicarbonate, potassium bicarbonate, ammonia bicarbonate, sodium hydroxide, potassium hydroxide, preferably urea.

Furthermore, said heating temperature is 60-150 °C, preferably 90-110 °C. The reaction time is 6-48 h, preferably 20-28 h.

The number of moles of the alkali precursor is 1-10 times of the total number of moles of the metals (the total number of moles of nickel and aluminium), preferably 2-4 times.

The high temperature calcination serves to provide metal oxides, which function by P-doped metal oxides instead of conventional metal phosphides avoiding the problem of deactivation due to carbon deposition or phase transformation in the nickel phosphide phase. As a preferred embodiment, said high temperature aerobic calcination is carried out at a temperature of 500-1000 °C, preferably 750-850 °C. Said high temperature calcination period is 0.5-12 h, preferably 1-3 h; and the heating rate is 1-20 °C/min.

In one or more embodiments of the present invention, said phosphorus source is a substance containing P element, comprising but not limited to red phosphorus, phosphine, phosphoric acid, phosphates, hypophosphates, phosphonic acid, hypophosphonic acid and the derivatives thereof, organophosphine and the derivatives thereof, phosphorus-containing organic substances and phosphorus-containing species resulting from the thermal decomposition thereof.

Furthermore, said phosphorus doping is carried out at a temperature of 250-425 °C, within which temperature range nickel phosphide phase will not be formed. Preferable temperature is 325-375 °C, more preferably 350 °C.

Said doping time is 1- 24 h, preferably 2-6 h.

The process of mixing and heating the phosphorus source and the nickel-aluminium oxide is a process of doping P into the nickel-aluminium oxide, i.e. the process of constructing the NiP interactions. Therefore, the molar ratio of P to Ni is essential for the construction of the Ni-P interactions. As a preferred embodiment, the phosphorus source is mixed with the nickel-aluminium oxide in a molar ratio of P:Ni = 1:1 - 30:1, preferably a molar ratio of 10:1 - 20:1.

In a further embodiment, the heating method for doping the phosphorus of said phosphorus source into the nickel-aluminium-based oxide may be any one of a separated heating, a mixed heating, and a solvothermal method.

Separated heating method: A certain amount of nickel-aluminium oxide is weighted, and then a phosphorus precursor of a given molar ratio is weighted. The two substances are separately placed and heated to a certain temperature under the protection of an inert gas or in vacuum, and then the phosphorus doping is allowed to occur.

Mixed heating method: A certain amount of nickel-aluminium oxide is weighted, and then a phosphorus precursor of a given molar ratio is weighted. The above two substances are physically mixed and heated to a certain temperature under the protection of an inert gas or in a vacuum to allow phosphorus doping to take place.

Solvothermal method: a certain amount of nickel-aluminium oxide is weighed, and then a phosphorus precursor of a given molar ratio is weighed. The two substances are dispersed in a solvent and heated to a certain temperature under the protection of an inert gas or in a vacuum to allow phosphorus doping to take place.

In one or more embodiments of the present invention, after doping the phosphorus into the nickel-aluminium oxide, the resulting material is passivated with an oxygen-containing gas mixture.

In a further embodiment, said oxygen-containing gas mixture is an O₂/Ar mixture, wherein the volume fraction of O₂ is 0.5%.

A second aspect of the present invention is to provide a phosphorus-doped nickel-aluminium oxide obtained by the above-mentioned preparation method.

A third aspect of the present invention is to provide a use of the above phosphorus-doped nickel-aluminium oxide in catalytic hydrodeoxygenation reactions.

Said catalytic hydrodeoxygenation reaction is carried out at a temperature of 300-400 °C, a hydrogen pressure of 2-5 MPa, a molar ratio of hydrogen/substrate of 5-100, a mass air velocity of the feed of 0.01-100 h⁻¹ and, if necessary, an organic solvent not involved in the reaction can be used as reaction medium.

Furthermore, the reaction substrate in the catalytic hydrodeoxygenation reaction is an oxygen-containing organic molecule.

In a further embodiment, the oxygen-containing organic molecule is one or more of an alcohol, an acid, a ketone, an aldehyde, a phenol, an ester, a furan, a plant oil, and an animal fat;

Specifically, the oxygenated organic molecules comprise, but are not limited to: palm oil, gutter oil, coconut oil, castor oil, coffee seed oil, cottonseed oil, rap oil, microalgae oil, laurate, palmitate, oleate, stearate, guaiacol, phenol, furfural, 5-hydroxymethylfurfural, aliphatic alcohols, aliphatic aldehydes, aliphatic acids, and aliphatic ketones.

In order to enable a person skilled in the art to have a thorough understanding of the technical solutions of the present invention, the technical solutions of the invention will be illustrated in detail in the following by presenting specific inventive examples and comparative examples.

### Example 1

Preparation of a phosphorus-doped nickel-aluminium oxide (NiAI-800-P350):
(a) 9 mmol Ni(NO₃)₂·6H₂O, 3 mmol Al(NO₃)₃·9H₂O, and 27 mmol urea were dissolved in 90 mL of deionized water. The mixture was stirred until a clear solution was obtained and then transferred into a polytetrafluoroethylene hydrothermal reactor, which was placed in a drying oven to allow the reaction to proceed at 100 °C for 24 h;
(b) At the end of the hydrothermal reaction, a hydrotalcite-like precipitate was obtained, which was separated by filtration, washed 3-5 times with water and dried in an oven at 80 °C for 12 h;
(c) The dried sample was ground until a fine, uniform powder was obtained. It was then placed in a muffle furnace for aerobic calcination at a heating rate of 10 °C/min and a calcination temperature of 800 °C, at which temperature the sample was kept for 1 hour and then cooled to room temperature;
(d) 0.1 g of the resulting oxide and 1.0 g of sodium hypophosphite (NaH₂PO₂) were weighed and placed in a quartz tube, purged with argon (100 mL/min) for 30 min. The reaction system was evacuated and sealed, and then the temperature was raised to 350 °C and was held at this temperature for 2 h. Thereafter the system was cooled to room temperature, purged with O₂/Ar (0.5% O₂ by volume) for 1 hour to obtain the corresponding phosphorus-doped nickel-aluminium oxide, denoted as NiAl-800-P350.

### Example 2

Preparation of a phosphorus-doped nickel-aluminium oxide (NiAI-650-P350):
(a) 9 mmol Ni(NO₃)₂·6H₂O, 3 mmol Al(NO₃)₃·9H₂O, and 27 mmol urea were dissolved in 90 mL of deionized water. The mixture was stirred until a clear solution was obtained and then transferred into a polytetrafluoroethylene hydrothermal reactor, which was placed in a drying oven to allow the reaction to proceed at 100 °C for 24 h;
(b) At the end of the hydrothermal reaction, a hydrotalcite-like precipitate was obtained, which was separated by filtration, washed 3-5 times with water and dried in an oven at 80 °C for 12 h;
(c) The dried sample was ground until a fine, uniform powder was obtained. It was then placed in a muffle furnace for aerobic calcination at a heating rate of 10 °C/min and a calcination temperature of 650 °C, at which temperature the sample was kept for 1 hour and then cooled to room temperature;
(d) 0.1 g of the resulting oxide and 1.0 g of sodium hypophosphite (NaH₂PO₂) were weighed and placed in a quartz tube, purged with argon (100 mL/min) for 30 min. The reaction system was evacuated and sealed, and then the temperature was raised to 350 °C and was held at this temperature for 2 h. Thereafter the system was cooled to room temperature, purged with O₂/Ar (0.5% O₂ by volume) for 1 hour to obtain the corresponding phosphorus-doped nickel-aluminium oxide, denoted as NiAl-650-P350.

### Example 3

Preparation of a phosphorus-doped nickel-aluminium oxide (NiAI-500-P350):
a) 9 mmol Ni(NO₃)₂·6H₂O, 3 mmol Al(NO₃)₃·9H₂O, and 27 mmol urea were dissolved in 90 mL of deionized water. The mixture was stirred until a clear solution was obtained and then transferred into a polytetrafluoroethylene hydrothermal reactor, which was placed in a drying oven to allow the reaction to proceed at 100 °C for 24 h;
b) At the end of the hydrothermal reaction, a hydrotalcite-like precipitate was obtained, which was separated by filtration, washed 3-5 times with water and dried in an oven at 80 °C for 12 h;
c) The dried sample was ground until a fine, uniform powder was obtained. It was then placed in a muffle furnace for aerobic calcination at a heating rate of 10 °C/min and a calcination temperature of 500 °C, at which temperature the sample was kept for 1 hour and then cooled to room temperature;
d) 0.1 g of the resulting oxide and 1.0 g of sodium hypophosphite (NaH₂PO₂) were weighed and placed in a quartz tube, purged with argon (100 mL/min) for 30 min. The reaction system was evacuated and sealed, and then the temperature was raised to 350 °C and was held at this temperature for 2 hours. Thereafter the system was cooled to room temperature, purged with O₂/Ar (0.5% O₂ by volume) for 1 hour to obtain the corresponding phosphorus-doped nickel-aluminium oxide, denoted as NiAI-500-P350.

### Example 4

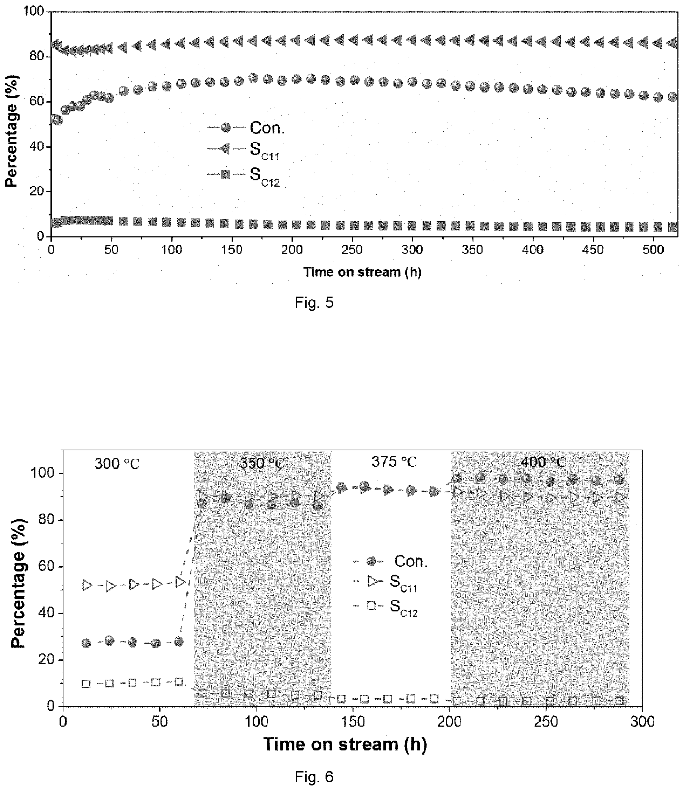

A phosphorus-doped nickel-aluminium oxide catalyzed hydrodeoxygenation of oil:
The NiAI-800-P350 obtained in Example 1 was used to catalyze the hydrodeoxygenation of methyl laurate for the preparation of undecane/dodecane. Firstly, 100 mg of catalyst was weighed and filled in a fixed-bed reactor, which was then charged with 3 Mpa of hydrogen. The reaction temperature was set to 350 °C and the flow rate of hydrogen was set to 110 mL/min. Methyl laurate was fed into the reactor at a flow rate of 0.055 mL/min. Samples were taken at regular intervals for detection, and the internal standard was tetrahydronaphthalene, and the reaction products were detected by gas chromatography and gas chromatography-mass spectrometry. The results are shown in Figure 5. The conversion of methyl laurate stabilized after 100 hours of operation and was maintained at about 65%, with no significant deactivation after 500 hours of continuous operation; the main products were undecane and dodecane throughout the reaction, and the selectivity was stable at about 87% and 5%, respectively.

### Example 5

Hydrodeoxygenation of oil catalyzed by the phosphorus-doped nickel-aluminium oxide (reaction temperature 300-400 °C):
The NiAI-800-P350 obtained in Example 1 was used to catalyze the hydrodeoxygenation of methyl laurate for the preparation of undecane/dodecane. Firstly, 100 mg of catalyst was weighted and filled in a fixed bed reactor, which was then charged with 3 Mpa of hydrogen. The initial reaction temperature was set to 300 °C and the flow rate of hydrogen was set at 110 mL/min. Methyl laurate was fed into the reactor at a flow rate of 0.055 mL/min. Samples were taken at regular intervals for detection. The internal standard was tetrahydronaphthalene, and the reaction products were detected by gas chromatography and gas chromatography-mass spectrometry. Afterwards, the reaction temperature was increased stepwise to 350, 375 and 400 °C to compare the effect of reaction temperature on the catalytic reaction.

The results are shown in Figure 6. The conversion of methyl laurate gradually increased with the increase of reaction temperature, reaching more than 97% at 400 °C. The selectivity of undecane and dodecane was about 90% and 2.5%, respectively.

### Example 6

Hydrodeoxygenation of methyl laurate catalyzed by the phosphorus-doped nickel-aluminium oxides:
The phosphorus-doped nickel aluminium oxides obtained in Example 2 and Example 3 were used to catalyze the hydrodeoxygenation of methyl laurate for the preparation of undecane/dodecane, respectively. First, 100 mg of catalyst was weighed and filled in a fixed-bed reactor, which was then charged with 3 Mpa of hydrogen. The reaction temperature was set to 350 °C and the flow rate of hydrogen was set to 110 mL/min. Samples were taken at regular intervals for detection, with the internal standard being tetrahydronaphthalene, and the reaction products were analyzed by gas chromatography and gas chromatography-mass spectrometry.

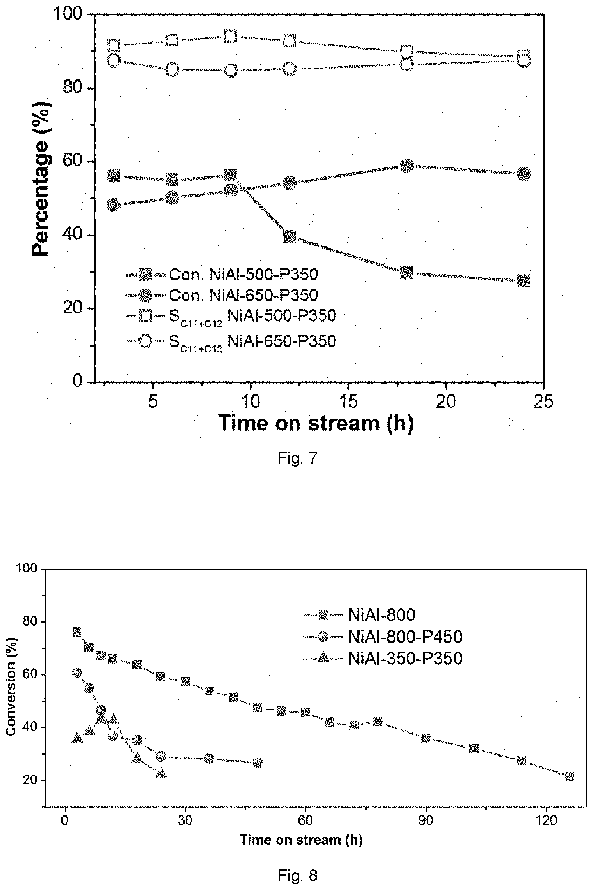

The results are shown in Figure 7. In the catalytic system of NiAI-650-P350 obtained in Example 2, the initial conversion of the reactants was 48% and was then gradually stable at about 57%, with the total selectivity of undecane and dodecane at about 90%. In the catalytic system of NiAI-500-P350 obtained in Example 3, the initial conversion of the reactants was 56% and then gradually decreased to 27% after 24 hours. The total selectivity of undecane and dodecane was stable at around 86%. These indicate that the catalytic activity and stability of the catalysts obtained at lower calcination temperatures are relatively poor.

### Example 7

The hydrodeoxygenation of methyl palmitate catalyzed by a phosphorus-doped nickel-aluminium oxides catalyzing:
The phosphorus-doped nickel-aluminium oxide obtained in Example 1 was used to catalyze the hydrodeoxygenation of methyl palmitate for the preparation of diesel chain hydrocarbons. Firstly, 100 mg of catalyst was weighed and filled in a fixed bed reactor, which was charged with 3 Mpa of hydrogen. The reaction temperature was set at 350 °C and the flow rate of hydrogen was set at 110 mL/min. A cyclohexane solution of methyl palmitate (30 wt.%) was fed into the reactor at a flow rate of 0.06 mL/min. Samples were taken at regular intervals for detection and the internal standard was tetrahydronaphthalene. The reaction products were detected by gas chromatography and gas chromatography-mass spectrometry.

The conversion of methyl palmitate was maintained at about 88%, and no significant deactivation was observed after 500 hours of continuous operation. The main products were pentadecane and cetane throughout the reaction, and the selectivity was stable at about 83% and 12%, respectively. The above data indicate that NiAI-800-P350 has excellent catalytic performance, especially in terms of anti-deactivation performance, and thus has great potential for industrialization.

### Example 8

Hydrodeoxygenation of palm oil catalyzed by the phosphorus-doped nickel-aluminium oxide:
The phosphorus-doped nickel-aluminium oxide obtained in Example 1 was used to catalyze the hydrodeoxygenation of palm oil for the preparation of diesel chain hydrocarbons.

Firstly, 100 mg of catalyst was weighed and filled in a fixed bed reactor, which was charged with 3 Mpa of hydrogen. The reaction temperature was set at 350 °C and the hydrogen flow rate was 110 mL/min. A cyclohexane solution of palm oil (30 wt.%) was fed into the reactor at a flow rate of 0.06 mL/min. Samples were taken at regular intervals for detection and the internal standard was tetrahydronaphthalene. The reaction products were analyzed by gas chromatography and gas chromatography-mass spectrometry.

The conversion of methyl palmitate was maintained at about 80% and no significant deactivation was observed after 150 hours of continuous operation. The main product was a C15-C18 chain hydrocarbon with a stable total selectivity of 85-90% throughout the reaction. The above data indicate that NiAI-800-P350 has excellent catalytic performance, especially in terms of anti-deactivation performance with great potential for industrialization.

### Comparative Example 1

Preparation of phosphorus-free nickel-aluminium oxide (NiAI-800):
a) 9 mmol Ni(NO₃)₂·6H₂O, 3 mmol Al(NO₃)₃·9H₂O and 27 mmol urea were dissolved in 90 mL deionized water and stirred until a clear solution was obtained. The solution was loaded into a polytetrafluoroethylene hydrothermal reactor and then placed in an oven at 100 °C to react for 24 hours;
b) At the end of the hydrothermal reaction, a hydrotalcite-like precipitate was obtained, which was separated by filtration, washed 3-5 times with water and dried in an oven at 80 °C for 12 hours;
c) The dried sample was ground until fine and homogeneous and then placed in a muffle furnace for aerobic calcination at a heating rate of 10 °C/min. The calcination was carried out at 800 °C for 1 hour. After cooling to room temperature, a phosphorus-free nickel aluminium oxide, denoted as NiAl-800, was obtained.

### Comparative Example 2

Preparation of a phosphorus-doped nickel-aluminium oxide (NiAI-350-P350):
a) 9 mmol Ni(NO₃)₂·6H₂O, 3 mmol Al(NO₃)·9H₂O and 27 mmol urea were dissolved in 90 mL deionized water and stirred until a clear solution was obtained. The solution was loaded into a polytetrafluoroethylene hydrothermal reactor and then placed in an oven at 100 °C to react for 24 hours;
b) At the end of the hydrothermal reaction, a hydrotalcite-like precipitate was obtained, separated by filtration, washed 3-5 times with water and dried in an oven at 80 °C for 12 h;
c) The dried sample was ground until fine and homogeneous and then placed in a muffle furnace for aerobic calcination at a heating rate of 10 °C/min. The calcination was carried out at 350 °C for 1 hour and then cooled to room temperature;
d) 0.1 g of the resulting oxide and 1.0 g of sodium hypophosphite (NaH₂PO₂) were weighed in a quartz tube. After purging with argon (100 mL/min) for 30 min, the reaction system was evacuated with a vacuum pump, sealed. The temperature was raised to 350 °C and maintained at this temperature for 2 hours. The system was then cooled to room temperature. Releasing the pressure and purging with O₂/Ar (0.5% O₂ by volume) for 1 hour to obtain the resulting phosphorus-doped Ni-Al oxide, denoted as NiAl-350-P350.

### Comparative Example 3

Preparation of nickel phosphide catalyst (NiAI-800-P450):
a) 9 mmol Ni(NO₃)₂·6H₂O, 3 mmol Al(NO₃)₃ 9H₂O, and 27 mmol urea were dissolved in 90 mL deionized water and stirred until a clear solution was obtained. The solution was loaded into a polytetrafluoroethylene hydrothermal reactor and then placed in an oven at 100 °C to react for 24 h;
b) At the end of the hydrothermal reaction, a hydrotalcite-like precipitate was obtained, which was separated by filtration, washed 3-5 times with water and dried in an oven at 80 °C for 12 hours;
c) The dried sample was ground until fine and homogeneous and then placed in a muffle furnace for aerobic calcination at a heating rate of 10 °C/min. The calcination was carried out at 800 °C for 1 hour and then cooled to room temperature;
d) 0.1 g of the resulting oxide and 1.0 g of sodium hypophosphite (NaH₂PO₂) were weighed in a quartz tube, purged with argon (100 mL/min) for 30 min. The reaction system was evacuated and sealed. The temperature was raised to 450 °C and maintained for 2 h before cooling to room temperature. Releasing the pressure and purging with O₂/Ar (0.5% O₂ by volume) for 1 hour to obtain the resulting nickel phosphide catalyst, denoted as NiAl-800-P450.

### Comparative Example 4

Phosphorus-free nickel-aluminium oxide-catalyzed hydrodeoxygenation of oil:
The NiAI-800 obtained in Comparative Example 1 was used to catalyze the hydrodeoxygenation of methyl laurate for the preparation of undecane/dodecane.

Firstly, 100 mg of catalyst was weighed and filled in a fixed bed reactor. The reactor was charged with 3 Mpa of hydrogen. The reaction temperature was set at 350 °C and the flow rate of hydrogen was set at 110 mL/min. Methyl laurate was loaded into the reactor at a flow rate of 0.055 mL/min. Samples were taken at regular intervals for detection, with the internal standard being tetrahydronaphthalene, and the reaction products were detected by gas chromatography and gas chromatography-mass spectrometry.

The results are shown in Figure 8. In the catalytic system of NiAI-800 obtained in comparison to Example 1, the initial conversion of the reactants was 76%, after which it gradually decreased to 21% after 120 h. The catalyst was less stable.

### Comparative Example 5

Hydrodeoxygenation of oils catalyzed by phosphorus-doped nickel aluminium oxide:
The NiAI-350-P350 obtained in Comparative Example 2 was used to catalyze the hydrodeoxygenation of methyl laurate for the preparation of undecane/dodecane.

Firstly, 100 mg of catalyst was weighed and filled in a fixed-bed reactor. The reactor was charged with hydrogen to 3 Mpa, the reaction temperature was set at 350 °C, the flow rate of hydrogen was set at 110 mL/min and methyl laurate was introduced into the reactor at a flow rate of 0.055 mL/min. Samples were taken at regular intervals for testing, with the internal standard being tetrahydronaphthalene, and the reaction products were detected by gas chromatography and gas chromatography-mass spectrometry.

The results are shown in Figure 8. In the catalytic system of NiAI-500-P350 obtained in Comparative Example 3, the initial conversion of the reactants was only 37% and the stability was very poor, dropping to 22% after 24 hours.

### Comparative Example 6

Hydrodeoxygenation of oils catalyzed by nickel phosphide catalysts:
The NiAI-800-P450 obtained in Comparative Example 3 was used to catalyze the hydrodeoxygenation of methyl laurate for the preparation of undecane/dodecane.

Firstly, 100 mg of catalyst was weighed and filled in a fixed-bed reactor. The reactor was charged with 3 Mpa of hydrogen to, the reaction temperature was set at 350 °C and the flow rate of hydrogen was set at 110 mL/min. Methyl laurate was introduced into the reactor at a flow rate of 0.055 mL/min. Samples were taken at regular intervals for detection, with the internal standard being tetrahydronaphthalene, and the reaction products were detected by gas chromatography and gas chromatography-mass spectrometry.

The results are shown in Figure 8. In the catalytic system of NiAI-800-P450 obtained in Comparative Example 3, the initial conversion of the reactants was 61%, after which it gradually decreased to 27% after 48 h indicating that the stability of the catalyst was poor.

The above description is only a preferred embodiment of the present invention and is not intended to limit the invention, which is open to various changes and variations for those skilled in the art. Any modification, equivalent substitution, improvement, etc. made within the spirit and principles of the present invention shall be included within the scope of protection of the present invention.

## Claims

1. A method for the preparation of phosphorus-doped nickel-aluminium oxide, **characterized in that** a nickel-aluminium-based layered double hydroxide is subjected to high temperature aerobic calcination to provide a nickel-aluminium oxide, said nickel-aluminium oxide is then mixed with a phosphorus source and heated under protection of an inert gas or in vacuum to dope phosphorus into the nickel-aluminium oxide, whereby the phosphorus-doped nickel-aluminium oxide is obtained.

2. The preparation method according to claim 1, **characterized in that** the molar ratio of Ni to Al in the nickel-aluminium-based layered double hydroxide is 5:1-1:5, preferably 4:1-2:1;
wherein said nickel-aluminium-based layered double hydroxide is synthesized by one or more methods selected from hydrothermal synthesis, co-precipitation, ion exchange, and calcination-recovery, preferably hydrothermal synthesis.

3. The preparation method according to claim 2, **characterized in that** the process for preparing the nickel-aluminium oxides is hydrothermal synthesis, wherein the nickel precursor, the aluminium precursor and the alkali precursor are heated in an aqueous solution by means of a hydrothermal reactor, followed by washing and drying to provide a nickel-aluminium-based layered double hydroxide;
wherein said nickel precursor and aluminium precursor are selected from one or more of nitrate, acetate, halide, sulphate, respectively, preferably nickel nitrate and aluminium nitrate; the alkali precursor is selected from one or more of urea, cyclic hexamethylene tetramine, sodium bicarbonate, potassium bicarbonate, ammonia bicarbonate, sodium hydroxide, and potassium hydroxide, preferably urea;
wherein said heating temperature is 60-150 °C, preferably 90-110 °C;
wherein the reaction time is 6-48 h, preferably 20-28 h; and
wherein the amount of the base precursor in mol is 1-10 times, preferably 2-4 times, of the total amount of nickel and aluminium in mol.

4. The preparation method according to claim 1, **characterized in that** the high temperature aerobic calcination is carried out at 500-1000 °C, preferably 750-850 °C,
wherein the high temperature aerobic calcination time is 0.5-12 h, preferably 1-3 h, and
wherein the heating rate is 1-20 °C/min.

5. The preparation method according to claim 1, **characterized in that** said phosphorus source is a substance containing P, which is selected from but not limited to red phosphorus, phosphine, phosphoric acid and phosphates, hypophosphates, phosphonic acid, hypophosphonic acid and the derivatives thereof, organophosphine and the derivatives thereof, phosphorus-containing organic substances and phosphorus-containing species resulting from the thermal decomposition thereof,
or, wherein said phosphorus doping temperature is 250-425 °C, preferably at a temperature of 325-375 °C, more preferably at 350 °C;
wherein said doping time is 1-24 h, preferably 2-6 h.

6. The preparation method according to claim 1, **characterized in that** the phosphorus source is mixed with the nickel-aluminium oxide in a molar ratio of P:Ni = 1:1 - 30:1, wherein the molar ratio is preferably 10:1 - 20:1;
or, wherein the heating method for doping the phosphorus in said phosphorus source into the nickel-aluminium-based oxide is any one of the methods selected from separated heating method, the mixed heating method, and the solvothermal method.

7. The preparation method according to claim 1, **characterized in that** after doping the phosphorus into the nickel-aluminium oxide, the resulting material is passivated with an oxygen-containing gas mixture;
wherein said oxygen-containing gas mixture is an O₂/Ar mixture, and wherein the O₂ volume fraction is 0.5%.

8. Phosphorus-doped nickel-aluminium oxide obtained by the preparation method according to any one of claims 1-7.

9. Use of the phosphorus-doped nickel-aluminium oxide according to claim 8 in catalytic hydrodeoxygenation reactions.

10. Use according to claim 9, **characterized in that** said catalytic hydrodeoxygenation reaction is carried out at a temperature of 300-400°C, a hydrogen pressure of 2-5 MPa, a molar ratio of hydrogen/substrate of 5-100, a weight hourly space velocity of the feed of 0.01-100 h⁻¹ and, where necessary, an organic solvent not involved in the reaction is used as a reaction medium;
wherein the reaction substrate in the catalytic hydrodeoxygenation reaction is an oxygen-containing organic molecule;
wherein the oxygen-containing organic molecule(s) is/are one or more of an alcohol, an acid, a ketone, an aldehyde, a phenol, an ester, a furan, a plant oil or an animal fat;
specifically, the oxygen-containing organic molecule comprises, but is not limited to palm oil, gutter oil, coconut oil, castor oil, coffee seed oil, cottonseed oil, rapeseed oil, microalgae oil, laurate, palmitate, oleate, stearate, guaiacol, phenol, furfural, 5-hydroxymethylfurfural, aliphatic alcohol, aliphatic aldehyde, aliphatic acid, aliphatic ketone.
